# EUROPEAN PATENT SPECIFICATION

(11) **EP 3 826 775 B1**
(45) Date of publication and mention of the grant of the patent: **11.02.2026**
(21) Application number: 19841650.5
(22) Date of filing: 24.07.2019
(51) Int. Cl.: B05B 17/06, A61M 11/00, A61M 15/00

(54) **NEBULIZER**
ZERSTÄUBER
NÉBULISEUR

(30) Priority: 24.07.2018 AU 2018902671
(43) Date of publication of application: 02.06.2021
(73) Proprietor: Monash University, Clayton, Victoria 3800 (AU)
(72) Inventor: ALAN, Tuncay, Clayton, Victoria 3800 (AU); NEILD, Adrian, Clayton, Victoria 3800 (AU); HOAI AN LE, Nguyen, Clayton, Victoria 3800 (AU); BRENKER, Jason, Clayton, Victoria 3800 (AU)
(74) Representative: Berggren Oy
(86) International application number: PCT/AU2019/050778
(87) International publication number: WO 2020/019030

(56) References cited:
- EP-A1- 0 923 957
- WO-A2-2012/026963
- JP-A- 2004 226 365
- JP-A- 2012 148 256
- JP-A- H07 328 503
- US-A1- 2007 158 459
- US-A1- 2017 178 884
- US-B2- 8 967 493
- US-B2- 9 358 568

## Description

### Technical Field

The present invention relates to a nebulizer that is capable of generating very small droplets for use in various fields such as thin film deposition, pulmonary drug delivery and mass spectrometry. It is expected that other fields will have use for the nebulizer of the invention.

### Background of Invention

The discussion of the background to the invention that follows is intended to facilitate an understanding of the invention. However, it should be appreciated that the discussion is not an acknowledgement or admission that any aspect of the discussion was part of the common general knowledge as at the priority date of the application.

Various different forms or nebulizers exist already and fall generally into four different groups, comprising 1) Jet nebulizers, 2) Ultrasonic nebulizers, 3) Mesh nebulizers and 4) Surface Acoustic Wave atomizers. These are discussed separately below.

### Jet Nebulizers

Jet nebulizers rely on a source of constant gas flow from either a compressor or central compressive gas supply to push a liquid dose through a small aperture to generate small aerosol droplets. Jet nebulizers are advantageously relatively cheap and of low complexity, but disadvantageously, they have low efficiency and require the separate gas source. In respect of efficiency and using the example of pulmonary drug delivery, less than 10% of the droplets created by a Jet nebulizer will be fit for inhalation delivery. The remaining droplets are recycled in a liquid reservoir that forms part of the nebulizer. The recycled liquid is available for return to the aperture for the next use of the nebulizer.

An improved nebulizer would dispense with the need for a compressor or central compressive gas supply and would have increased efficiency in terms of useful droplet generation.

### Ultrasonic Nebulizer

Ultrasonic nebulizers employ a piezoelectric (PZT) material to vibrate a liquid and generate droplets from it. There are two different arrangements that are usually employed in which in the first arrangement, the **PZT** is placed in direct contact with the liquid to be atomised and in the second arrangement, a liquid medium is positioned between the **PZT** and the liquid to be atomised and the respective liquids are separated by a membrane. Both of these arrangements are inefficient in only producing small quantities of usable atomised droplets so that both of these arrangements still require a recycling facility for the larger droplets to be accumulated for subsequent further atomisation.

Further disadvantages with this form of nebulizer are that it cannot aerosolise viscous solutions, while the liquid to be atomised is exposed heat generated by the PZT as a result of the close proximity of the PZT to the liquid, particularly in the first arrangement where there is direct contact between the PZT and the liquid. That exposure can result in degradation of liquids that are heat-sensitive. A still further disadvantage with this form of nebulizer is that the amount of liquid that can be atomised is smaller than the initial volume that is provided to the nebulizer, which results in a residual volume of liquid following completion of atomisation.

An improved nebulizer would have a reduced residual volume of liquid and reduced application of heat to the liquid being atomised.

### Mesh Nebulizer

Mesh nebulizers have been developed more recently and provide, in the view of the inventor, the most advanced current approach in liquid atomization technology. Mesh nebulizers employ a porous mesh through which a liquid is passed to form very small droplets. A typical mesh has thousands of very small pores with the pore size dictating the droplet size that is produced. The pores can be conical so that a wide inlet is provided which narrows to a narrow outlet.

Mesh nebulizers are categorised into two major groups, being static mesh and vibrating mesh. There is a simple difference between the two forms of mesh nebulizer and that is in the positioning of the PZT actuator of these devices. In a vibrating mesh nebulizer, the PZT actuator is placed in direct contact with the mesh, whereas in a static mesh nebulizer, the PZT actuator is positioned spaced from the mesh.

A drawback with the mesh nebulizer devices is that the mesh component is expensive given the large number of very small pores required and is thus not realistically disposable (because of the expense). This makes the overall device expensive. In addition, daily or weekly cleaning is necessary to prevent the small pores from becoming blocked, such as from crystallisation of the samples being atomised. Even with regular cleaning, the mesh component typically needs to be replaced every six months.

### Surface Acoustic Wave Nebulizer

Surface acoustic wave (SAW) nebulizers utilize a PZT substrate to excite a liquid sample and to atomize droplets via capillary wave breakup.

SAW nebulizers can advantageously control the size of the droplets produced, but disadvantageously they operate at low throughputs and can cause excessive heating of the samples to be atomised.

US 2007/0158459 A1 discloses a spray generator utilising a vibrating surface including hollows in the form of grooves or slots. JP 2004-226365 A discloses a nebulizer having a substrate including a concealed flow path between reservoirs. A nebulizer according to the preamble of claim 1 and employing a substrate including an open channel in which fluid to be atomised is contained is disclosed in JP 2012-148256 A. The present invention is intended to provide a nebulizer that overcomes drawbacks or difficulties associated with the prior art, including but not limited to the drawbacks or difficulties associated with the prior art discussed above.

### Summary of Invention

According to the present invention there is provided a nebulizer characterized in claim 1.

A nebulizer according to the invention employs a channel as the receptacle in which fluid to be atomised is contained. This distinguishes the invention from prior art nebulizers which provide fluid as a large body that is progressively atomised. In contrast, in a nebulizer according to the invention, fluid is fed or drawn into a channel and only that fluid within the channel is subject to atomisation by the high frequency vibration that is generated by the vibration generator and that transmits through the substrate to the channel. The forms of each of the prior art nebulizers discussed as prior art herein as known to the inventor employ a large body of liquid that is atomised from that large body. Those nebulizers do not feed a channel from a body of liquid. The feed can, for example, be by capillary action upon excitation of the substrate by the vibration generator.

Testing of nebulizers according to the invention has established that the variation in droplet size of the droplets that are produced can be controlled or tuned so that the level of variability is very low and the consistency is very good. In some forms of the invention, the size of the particles can be tuned on-demand by changing the frequency of actuation of the vibration generator. This has advantageous outcomes for aerosol drug delivery into the lungs for example, as the size of delivered droplets determines where in the lung the droplets are deposited as well as influencing the therapeutic effectiveness of the drug.

From measurements of the droplet size relative to the driving frequency of the high frequency vibration, as well as imagery that has been observed of the fluid/air interface, the physical mechanism of the atomising effect of nebulizers according to the invention is considered to involve capillary waves forming at the fluid/air interface at the top or opening of the channel or channels formed in the substrate. The periodicity of this interface is set by the driving frequency. This in turn regulates the size of droplets produced. Thus by using a vibration generator such as a piezoelectric actuator which is capable of exciting at or to a range of or various frequencies, different sizes of droplets can be produced from the same nebulizer. Physical properties affecting capillary wavelength at the fluid/air interface include 1) the density of the two fluids in contact, 2) the surface tension and 3) the driving wavelength.

A nebulizer according to the invention can employ a linear channel of any suitable form or geometry. Advantageous results have been achieved with a channel having a high aspect ratio in which the depth of the channel is much greater than the width. Examples of channels that are expected to be suitable for use in the invention and that have a high aspect ratio, include channels in which the aspect ratio ranges from 7:1 to 15:1.

The linear channel that is employed in the invention can be a microscopic channel consistent with the requirement to generate microscopic particles. In some forms of the invention, the opening of the channel through the upper surface of the substrate has a width transverse to the length of about 1.5 micrometres. In other forms of the invention, the opening of the channel can have a width of about 20 micrometres. Other widths that have been employed in testing to date include 3, 5 and 10 micrometres. Where the channel that is employed is a high aspect ratio channel, the depth of the channel can thus be from 10.5 micrometres for a channel that has an aspect ratio of 7:1 and a channel opening of 1.5 micrometres and up to a 300 micrometre depth for a channel that has aspect ratio of 15:1 and a channel opening of 20 micrometres.

The linear channel that is employed in the invention can be formed in any suitable manner. Because it will normally be formed in microscopic dimensions, not all ordinary fabrication methods will be available. Early prototypes have employed etching technology, in particular deep reactive ion etching to form the channel.

The substrate can be formed from any suitable material. The substrate is a solid substrate and is preferably a rigid substrate. Early prototypes have employed a substrate formed from silicon which is readily available, cheap, easily fabricated and advantageously, is inert to most biological and chemical components. Moreover, because silicon is cheap and readily fabricated to form the linear channel, a further benefit of the use of silicon for the substrate is that the substrate can be disposable. The main benefit of this is that the use of a disposable substrate eliminates the need for the substrate to be cleaned and/or sterilised between uses. Cleaning is required in prior art nebulizers mainly to clear clogged or blocked pores through which atomisation of liquid takes place, while sterilisation is required to prevent contamination of one liquid by the next. The absence of pores in the present invention is considered to provide significant advantages over the prior art.

Alternative materials for the substrate include glass, fused silica and Lithium Niobate for example.

The surface of the channel can be treated if required and depending on the type of fluid to be atomised. In the invention, the surface of the channel is treated to render it hydrophilic. That surface treatment can be achieved in a silicon substrate by the application of O₂ plasma to the channel surface. Other forms of surface treatment however could be employed, particularly if the substrate is other than silicon.

A single linear channel can be formed in the substrate, or multiple channels can be formed. The use of multiple channels enables the output of the nebulizer according to the invention to be increased. Where multiple channels are formed, these can be formed in a parallel array. Two channels can be formed parallel to each other or three or more channels can be formed in parallel formation. Alternatively, the two or more channels can be formed other than in a parallel formation and could, for example be formed perpendicular to each other, such as in a T or L formation for two channels, and in a C or square/rectangular formation for three or four channels. Other formations and other numbers of channels can be employed. Equally, where multiple channels are employed, the respective channels can be of the same dimensions or of different dimensions. The dimensions of the respective channels might differ as a result of their different proximity and/or orientation relative to the vibration generator.

The vibration generator can have any suitable form however in current forms of the invention, the vibration generator is a piezoelectric actuator that employs a rectangular piezoelectric block that is actuated or excited electrically. The use of a piezoelectric actuator permits nebulizers according to the invention to have a very small footprint while also allowing the vibrations that are generated in the solid substrate to have a high frequency. Early prototypes have generated actuation vibrations in a frequency range from 1.5 to 4.2MHz, although the expected frequency range is from about 0.5 to 5MHz. Even higher frequencies, such as in the range of 5 to 20 MHz or even much higher, could be generated and would be expected to provide beneficial results.

A vibration generator can be placed on the upper surface of the substrate through which the channel or channels open. The vibration generator can be spaced from the channel or channels any suitable distance. The distance can be selected to ensure that heating of the fluid being atomised by the vibration generator is minimised or is negligible. Also, the vibration generator can be pulsed on and off rather than being left on continuously to further limit heat transfer to the fluid being atomised. This distinguishes a nebulizer according to the invention from some of the prior art discussed herein in which heating of the fluid being atomised is a problem or disadvantage.

In early prototypes, a single linear channel has extended across the substrate and the vibration generator, in the form of a rectangular piezoelectric block, has been positioned to generate high frequency vibrations that transmit through the substrate. In these early prototypes, the vibrations have mainly transmitted through the substrate perpendicular to the channel, however parallel transmission can also be employed.

In alternative arrangements, the vibration generator can be placed on the lower or underneath surface of the substrate opposite to which the channel or channels open. In one particular form of the invention, the vibration generator can be placed directly below the closed base of the channel or channels, so that vibrations transmit through the thickness of the substrate through to the base of the channel and thereafter through to the fluid within the channel or channels. In this arrangement, the thickness of the substrate between the lower or underneath surface of the substrate and the base of the channel or channels might be greater than the thickness of the substrate between the lower or underneath surface and the upper surface when the vibration generator is placed on the upper surface of the substrate.

Indeed, the thickness of the substrate between the upper and lower surfaces can vary if required. In early prototypes, the thickness of the substrate between the upper and lower surfaces has been constant and in the range of 200 to 525 micrometres. In these prototypes, the substrate has been of a rectangular configuration although other configurations such as square, circular or other configuration remains within the scope of the present invention.

If the vibration generator is chosen to be a piezoelectric actuator, then any suitable form of that actuator can be employed. The piezoelectric actuator will be electrically activated or excited and this could be by mains electrical power, or more preferably, by battery power. Persons skilled in the art of the prior art nebulizers discussed herein will be familiar with suitable piezoelectric actuators and how they are set up to generate vibration.

A piezoelectric actuator can be attached to the substrate in any suitable manner such as by acoustic coupling fluids or adhesives.

As indicated above, the substrate can be a disposable substrate. In contrast, the piezoelectric actuator is preferably not disposable given it has a significant cost relative to the substrate and so the actuator preferably is transferable between substrates and so the manner of attaching the piezoelectric actuator to the substrate preferably can accommodate this.

The linear channel formed in the substrate can extend fully across the substrate from one side to the other and open through the side edges of the substrate. Alternatively, one end of the linear channel can open through one side edge of the substrate and the other and opposite end can terminate within the substrate and so have a blind end. Still alternatively, both ends of the linear channel can terminate within the substrate and so both ends can be blind.

The linear channel is fed fluid from the feeding facility. The feeding facility can have any suitable form and in one form, includes a reservoir into which fluid is fed for subsequent passage or travel into the channel. The reservoir can be formed in the substrate. The reservoir can be formed in direct communication with the channel, for example with one end of the channel. That is, the channel can open directly into the reservoir. The feeding facility can feed the reservoir from which fluid can enter the channel for atomisation. The reservoir can have any suitable shape and configuration and in some forms it has a generally circular periphery that opens through the upper surface of the substrate and is generally spherical below the upper surface. The depth of the reservoir can be about the same depth as the channel, or it can be of a greater or lesser depth.

A single reservoir can feed multiple channels so that more than one channel extends into direct communication with the reservoir. For example, a pair of intersecting channels that form a cross could be fed by a reservoir that is located at the intersection of the channels. Alternatively, multiple reservoirs can be provided to feed multiple channels.

A reservoir can be positioned at one end of a linear channel as described above, or it can be positioned elsewhere within the channel. A reservoir could be positioned centrally of a channel for example so that fluid can be fed into the channel from either side or opposite sides of the channel. Alternatively, a pair of channels that are positioned perpendicular to each other can both intersect with and thus be in direct communication with a reservoir. A pair of reservoirs can be employed with one reservoir being positioned at one end of a channel and a second reservoir positioned at the opposite end.

The feeding facility can comprise just the reservoir or reservoirs discussed above, so that in use, fluid is delivered into the reservoir or reservoirs and thereafter the nebulizer of the invention can be operated to atomise the fluid. Atomisation can continue until all of the fluid is atomised or until a satisfactory amount of fluid has been atomised. Where the substrate of the nebulizer is disposable, any excess fluid can be disposed of as well, or where the substrate of the nebulizer is to be cleaned or sterilized, any excess fluid will be removed in that process.

However, the feeding facility can include further structure and can for example include a feed conduit or tube that facilitates feed of fluid into the reservoir or reservoirs. Alternatively, the feeding facility can include a feed conduit or tube that facilitates feed of fluid directly into the channel or channels, so that a reservoir formed in the substrate is not required. In this form of the invention, the feeding facility can still include a reservoir, but the reservoir is not formed in the substrate, but is separate from the substrate. In this latter respect, the reservoir can be an external reservoir and for example, such a reservoir could be in the form of a container such as a blister pack or the like. Another form of external reservoir could be a vial. In each case, the blister pack or vial could already be filled with a fluid to be atomised and can be connected directly or indirectly to the channel. A suitable connection to connect between the blister pack or vial and the nebulizer can form part of the nebulizer.

Alternatively, the feeding facility can include a wicking arrangement to wick fluid from a reservoir or fluid supply, which is either part of the substrate or is separate from the substrate.

The feeding facility can be set up so that a particular dose of a particular fluid can be delivered or made available to the channel or channels. Alternatively, the arrangement can be such that once the reservoir or the channel is filled appropriately, further feed terminates. The feeding facility can be arranged to be self-replenishing so that upon fluid in the channel or reservoir being exhausted, or reduced to a certain level, the feeding facility can replenish the channel or reservoir. Alternatively, the feeding facility can be arranged to replenish the channel or reservoir constantly as fluid in the channel is atomised so that the level of fluid in the channel or reservoir remains generally constant. The feeding facility can thus also remove the need for residual fluid that is not atomised to be recycled. This is a significant benefit.

The entry or passage of fluid into the channel or channels can be by any suitable mechanism, and advantageously, capillary action or flow can be employed. Thus, in some forms of the invention, a reservoir can supply a channel by capillary action, so that as fluid within the channel is atomised and drawn out of the channel, fluid within the reservoir flows into the channel automatically and without external input, such as by pumping. This arrangement can be used provide an atomised dosage, by the reservoir including a set amount of fluid representing a single dose and the fluid flowing under capillary action into the channel from the reservoir until the fluid within the reservoir is exhausted or is reduced to a level at which capillary flow ceases or terminates.

Nebulizers according to the invention can operate with low heat waste as the liquid medium is separated from and therefore not in contact with the vibration generator, usually a piezoelectric actuator. Nebulizers according to the invention have a channel spaced from the vibration generator and fluid is drawn into the channel from a feeding facility. Early prototypes of the invention have advantageously atomised highly monodisperse micro droplets with diameters ranging from 4.3 to 6.5 micrometres with a size variation of about 0.25 micrometres. The stable nature of the invention and the ability to provide consistent fluid feed means that the droplet size produced can be tuned or specified and consistently provided.

According to an example, the solid substrate is housed within a housing, the housing defining a chamber and having an outlet to the chamber for the egress of atomised fluid particles from within the chamber, the solid substrate being within the chamber of the housing. The components of the nebulizer described above are housed within the housing to form the nebulizer. The housing defines the chamber within which atomised droplets or fluid particles accumulate while the outlet of the chamber allows for a user of the nebulizer to draw the atomised droplets out of the chamber, such as for inhalation. The outlet can be appropriately shaped and sized for inhalation purposes or it can be arranged for the attachment of an inhalation device, such as a mouthpiece.

Where the invention is used for non-medical applications, such as for thin film deposition or mass spectrometry, the housing can be different, or it can be omitted entirely.

In the above form of the invention, the atomised fluid is not captured within the chamber of a housing, but rather, is otherwise captured or directed as required. A nebulizer according to this form of the invention can have all of the features described above in relation to the earlier form of the invention in which a housing is provided.

In a nebulizer according to the invention, the atomised droplet size can be tuned by modifying channel geometry, and actuation frequency and amplitude. A key advantage of the present invention is the ability to alter the design of the substrate, including the dimensions and geometry of the channel or channels and the spacing of the vibration generator from the channel or channels, which would be able to provide different types of droplets (in term of physical size and chemical composition). Different channel design and surface modification are important to this advantage. Unlike other commercially available nebulizers, especially in comparison to vibrating mesh nebulizers, the present invention will enable users to easily change the substrate and thus change the characteristics of the atomized fluid. That is, both the structure of a channel of the substrate and the location of the vibration generator relative to a channel of the substrate can be changed by changing the substrate of the nebulizer.

The invention advantageously provides a simple and cheap nebulizer for the generation of highly monodispersed aerosol particles.

### Brief Description of Drawings

In order that the invention may be more fully understood, some embodiments will now be described with reference to the figures in which:
Figure 1 is a perspective view of a nebulizer according to one embodiment of the invention.
Figure 2 is a side view of a nebulizer according to another embodiment of the invention.
Figure 3 is a photograph in cross-sectional side view of a prototype nebulizer according to the invention.
Figure 4 is a graph created from prototype testing in relation to nebulizers according to the invention that are excited at different frequencies.
Figure 5 graphically illustrates the typical size distribution of droplets for a 20 µm wide, 200 µm deep channel in a substrate that is excited at 675 MHz.
Figures 6(a) to 6(c) are a series of still images taken from high speed imagery looking down on 13.75 µm wide channels etched into a a silicon wafer during nebulisation.
Figure 7 is a still image of the atomized droplets produced in a nebulizer that has a channel that is 20 µm wide and 200 µm deep that is excited at 675 MHz.

### Detailed Description

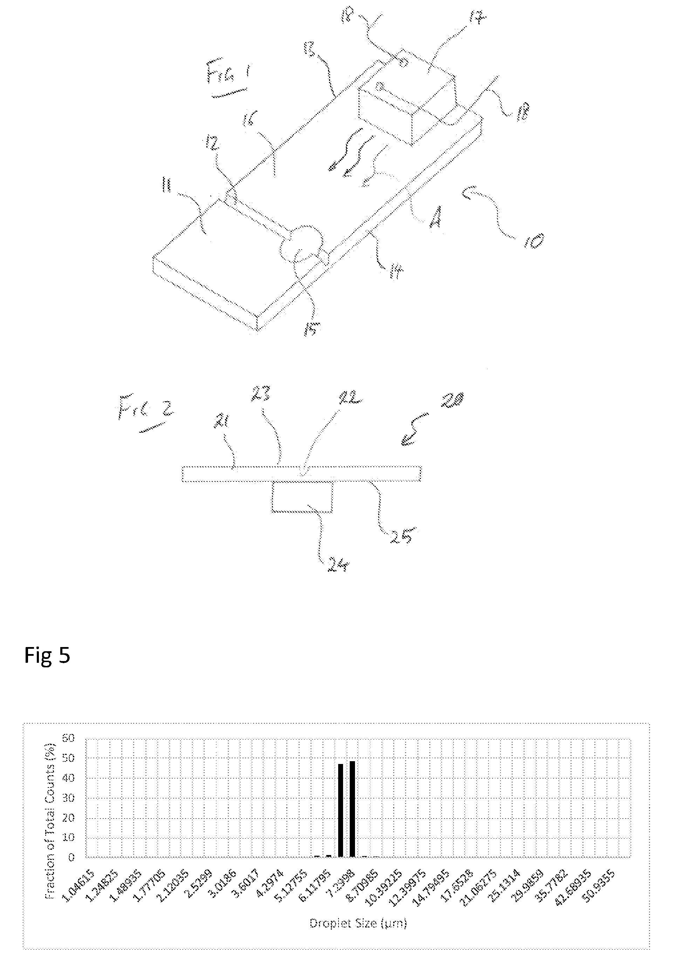

Figure 1 illustrates a nebulizer 10 according to one embodiment of the present invention. The nebulizer 10 is shown without a housing being depicted, so that the features of the nebulizer 10 can be easily shown. Accordingly, the nebulizer 10 of Figure 1 is formed from a substrate 11 that is rectangular in configuration and includes a linear channel 12 that extends laterally across the substrate 11 between respective long sides 13 and 14 of the substrate 11. It can be seen that the channel 12 extends through the long side 13 at one end, and extends into a generally circular reservoir 15 at the other end. The channel 12 extends for a short extent on the opposite side of the reservoir 15 to extend through the opposite long side 14. The channel 12 includes a closed base and opposite side walls and an opening that opens through the upper surface 16 of the substrate 11. The dimensions of the channel 12 are greatly exaggerated in Figure 1 for the purpose of illustration. As will be appreciated from the discussion earlier herein, the opening of the channel 12 through the upper surface 16 of the substrate 11 can have a width transverse to the length from about 1.5 micrometres to about 20 micrometres. The depth of the channel can be from about 10.5 micrometres up to about 300 micrometres.

A vibration generator 17 is mounted on the upper surface 16 of the substrate 11 at a position spaced from the channel 12 and is in the form of a piezoelectric actuator. The piezoelectric actuator is a piezoelectric block. The actuator 17 is connected in a normal manner to cables 18 that attach to separate electrodes of the actuator 17 and that supply electric current to the actuator 17 for the purpose of exciting the actuator 17. Upon the actuator 17 being excited, high frequency vibrations are generated that transmit through the substrate 11 in the direction towards the channel 12 as shown by the arrows A. The vibrations reach the channel 12 and cause atomisation of fluid within the channel 12.

Figure 2 illustrates an alternative arrangement of a nebulizer 20 according to another embodiment of the present invention. The nebulizer 20 is again shown without a housing being depicted. The nebulizer of Figure 2 is shown from the side and includes a square or rectangular substrate 21 and linear channel 22 that extends laterally across the substrate 21. The channel 22 is fed by a generally circular reservoir which is not shown, but which can be assumed to be the same as or similar to the reservoir 15 of Figure 1. Thus, the channel 22 extends through opposite sides of the substrate 21. The channel 22 includes a closed base and opposite side walls and an opening that opens through the upper surface 23 of the substrate 21. The dimensions of the channel 22 are greatly exaggerated in Figure 2 as they were in Figure 1 for the purpose of illustration.

A vibration generator 24 is mounted on the lower or underneath surface 25 of the substrate 21 at a position spaced from the closed base of the channel 22. The vibration generator 24 is again in the form of a piezoelectric actuator. The actuator 24 is connected in a normal manner to cables (not shown) that supply electric current to the actuator 24 for the purpose of exciting the actuator 24. Upon the actuator 24 being excited, high frequency vibrations are generated that transmit through the substrate 21 in the direction towards the channel 22. Obviously the distance shown for the vibrations to travel to reach the channel 22 is much shorter than illustrated in Figure 1, but the thickness of the substrate 21 can be selected to provide an appropriate distance for vibration propagation. In the same manner as Figure 1, the vibrations reach the channel 22 and cause atomisation of fluid within the channel 22.

The vibration generator 24 is placed directly below the closed base of the channel 22. In alternative arrangements, the vibration generator 24 can be offset from the channel 22 but still located on the underneath surface 25 of the substrate 21.

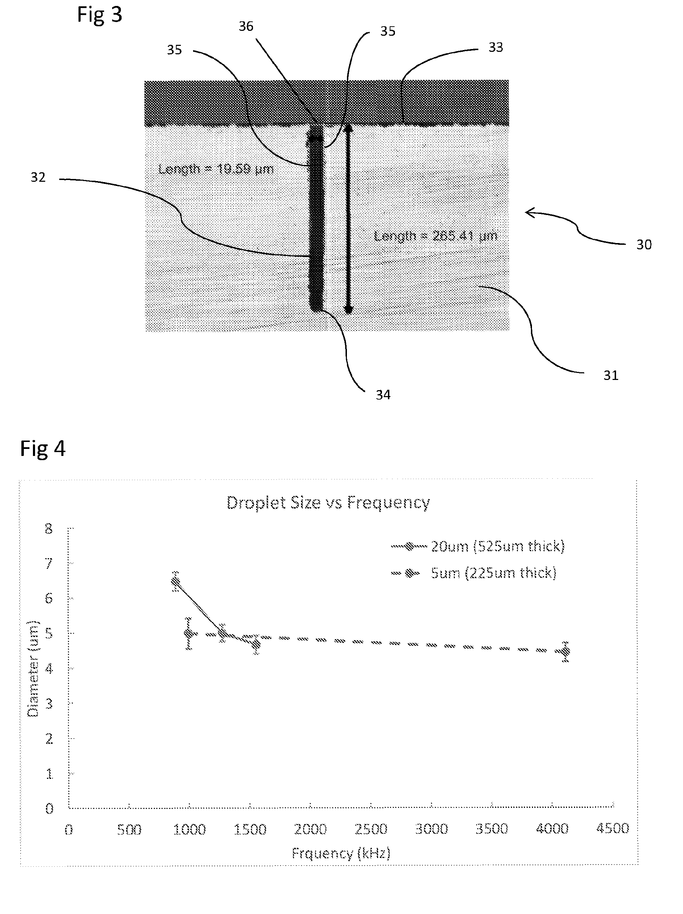

Figure 3 is a photograph taken with a microscope and illustrates an actual prototype nebulizer according to the invention which is shown in side cross-section. The nebulizer 30 has a substrate 31, a channel 32 and an upper surface 33. The dark section above the surface 33 is background and is not part of the nebulizer 30. The channel 32 has a closed base 34, opposite side walls 35 that are generally parallel and a lateral opening 36 that opens through the upper surface 33. The substrate 31 is a silicon wafer and the channel 32 is formed by deep reactive ion etching. The internal surface of the channel 32 has been rendered hydrophilic by the use of the application of O₂ plasma.

The dimensions of the channel 32 are given in Figure 3. Thus, the transverse width of the channel 32 is 19.59 µm, while the length or height of the channel 32 extending downwardly from the upper surface 33 to the close base 34, is 265.41 µm. These measurements will give context to the nebulizers 10 and 20 illustrated in Figures 1 and 2, in terms of dimensional size.

Figure 4 is a graph created from prototype testing in relation to nebulizers according to the invention that are excited at different frequencies. The Y-axis gives the diameter in µm of the droplet size that is produced, while the X-axis gives the vibration frequency that is applied to the substrate by the vibration generator. The graph shows that a range of the droplet sizes are produced at a particular frequency, The graph also illustrates the use of two different sized channels and substrate thicknesses, whereby the channel dimensions given is the lateral opening dimension of the channels through the upper surface of the substrate in which the channels are formed and the substrate dimension is the thickness of the substrate between the upper and lower surfaces of the substrate. Thus, the results relate to different channels having respective lateral openings of 20 µm and 5 µm and substrate thicknesses of 525 µm and 225 µm.

The graph shows that there is a highly advantageous result produced by the present invention. The graph for the 20 µm channel shows that the droplet size does not vary greatly at a particular frequency. For the 20 µm channel, at each of the approximate 1MHz, 1.3MHz and 1.6MHz points, the extent to which there is variation in droplet size is approximately the same and within the range of about 0.5 µm. For the 5 µm channel, at each of the approximate 1MHz and 4.15MHz points, the variation in droplet size is approximately between 0.5 µm and 0.9 µm. That level of variability is very low and the consistency is very good.

This result is that the mean diameters of the droplets that are produced by a nebulizer according to the present invention, can be maintained within a small range of different sizes. Thus, the particles are generally of the same size. This differs from nebulizers according to the prior art, in which there is much greater variation in the diameter of droplets produced. Thus, the present invention provides high levels of control over the mean diameter of the droplets produced. This is highly advantageous for aerosol drug delivery into the lungs, as the size of delivered droplets determines where in the lung the droplets are deposited as well as influencing the therapeutic effectiveness of the drug. As shown in Figure 4, the size of the particles can be tuned on-demand by changing the frequency of actuation.

As previously described, from measurements of the droplet size relative to the driving frequency of the high frequency vibration, as well as imagery that has been observed of the fluid/air interface, capillary waves form at the fluid/air interface at the opening of a channel formed in the substrate. The use of a vibration generator such as a piezoelectric actuator which is capable of exciting a substrate at a range of frequencies, different sizes of droplets can be produced from the same nebulizer. Physical properties affecting capillary wavelength at the fluid/air interface include 1) the density of the two fluids in contact, 2) the surface tension and 3) the driving wavelength. Figure 5 graphically illustrates the typical size distribution of droplets for a 20 µm wide, 200 µm deep channel in a substrate that is excited at 675 MHz.

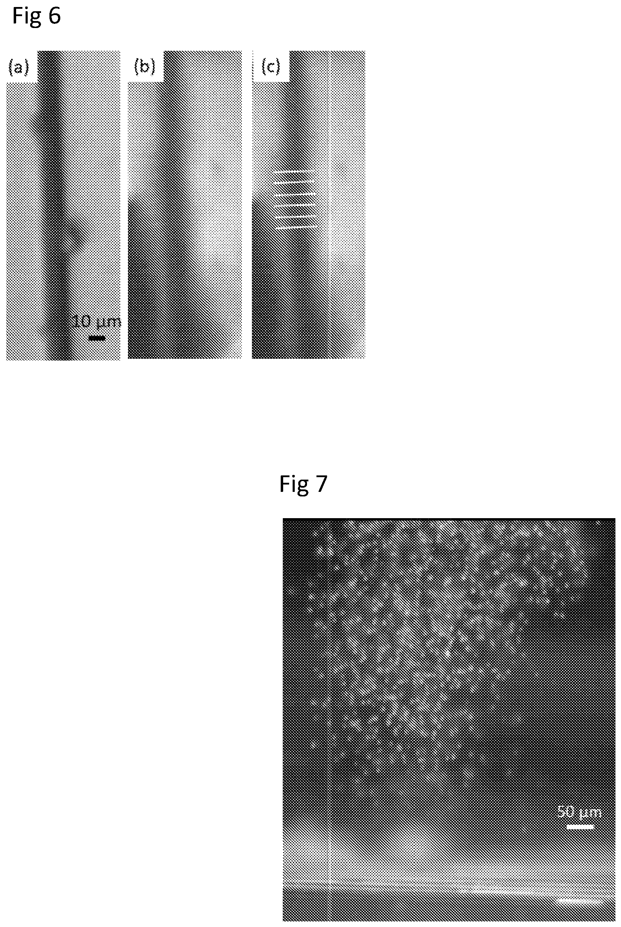

Figures 6(a) to 6(c) is a series of still images taken from high speed imagery looking down on 13.75 µm wide channels etched into a a silicon wafer during nebulisation. Figure 6(a) shows a water filled channel with no frequency generation. No capillary waves are apparent on the surface of the water. In Figure 6(b), a 675 MHz frequency generation has been applied to the substrate, creating capillary waves on surface of the water. In Figure 6(c) the wavelength of the capillary waves of Figure 6(b) are shown, and these have been measured to be approximately 9.5 µm.

Figure 7 is a still image of the atomized droplets produced in a nebulizer that has a channel that is 20 µm wide and 200 µm deep that is excited at 675 MHz. The nebulizer produces droplets approximately 7 µm in diameter. High speed imagery used to measure these droplets detected a droplet velocity about 0.6 - 1 m.s⁻¹ vertically.

Where any or all of the terms "comprise", "comprises", "comprised" or "comprising" are used in this specification (including the claims) they are to be interpreted as specifying the presence of the stated features, integers, steps or components, but not precluding the presence of one or more other features, integers, steps or components.

Those skilled in the art will appreciate that the invention described herein is susceptible to variations and modifications other than those specifically described. It is understood that the invention includes all such variations and modifications which fall within the enclosed claims.

## Claims

1. A nebulizer (10) comprising:
- a solid substrate (11),
- a linear channel (12) formed in the solid substrate (11) that has a closed base, opposite side walls and an opening that opens through an upper surface (16) of the solid substrate,
- a vibration generator (17) attached to the solid substrate (11) at a position spaced from the linear channel (12) for generating high frequency vibration that transmits through the solid substrate to the linear channel to atomise fluid within the linear channel,
- a feeding facility for feeding fluid to the linear channel (12),
**characterized by**
- the surface of the linear channel (12) being treated to render it hydrophilic,
- the linear channel (12) having a high aspect ratio in which the depth of the linear channel is much greater than the width of the linear channel, the depth of the linear channel being in the range of 10.5 micrometres for a linear channel (12) that has an aspect ratio of 7:1 and a linear channel opening of 1.5 micrometres and up to a 300 micrometre depth for a linear channel (12) that has an aspect ratio of 15:1 and a linear channel opening of 20 micrometres.

2. The nebulizer according to claim 1, the aspect ratio of the linear channel ranging from 7:1 to 15:1.

3. The nebulizer according to claim 1 or 2, the opening of the linear channel (12) through the upper surface (16) of the solid substrate having a width transverse to the length, the width being 1.5, 3, 5, 10 or 20 micrometres.

4. The nebulizer according to any one of claims 1 to 3, the solid substrate (11) being a rigid substrate, in particular being formed from silicon.

5. The nebulizer according to any one of claims 1 to 4, the surface of the linear channel (12) being treated to render it hydrophilic by the application of O₂ plasma to the linear channel surface.

6. The nebulizer according to any one of claims 1 to 5, including multiple linear channels (12) being formed in a parallel array or perpendicular to each other, or in a T or L formation for two linear channels, or in a C or square/rectangular formation for three or four linear channels.

7. The nebulizer according to any one of claims 1 to 6, the vibration generator (17) being a piezoelectric actuator that employs a piezoelectric disc that is actuated or excited electrically.

8. The nebulizer according to claim 7, the piezoelectric actuator generating actuation vibrations in a frequency range from 500KHz to 5MHz or from 5MHz to 20MHz.

9. The nebulizer according to any one of claims 1 to 8, the linear channel (12) extending fully across the solid substrate (11) from one side to the other and open through side edges of the solid substrate.

10. The nebulizer according to any one of claims 1 to 8, one end of the linear channel (12) opening through one side edge of the solid substrate (11) and the other and opposite end terminating within the solid substrate.

11. The nebulizer according to any one of claims 1 to 8, both ends of the linear channel (12) terminating within the solid substrate (11).

12. The nebulizer according to any one of claims 1 to 11, the feeding facility having a reservoir (15) into which fluid is fed for subsequent passage or travel into the linear channel (12).

13. The nebulizer according to claim 12, the reservoir (15) being formed in the solid substrate (11).

14. The nebulizer according to any one of claims 1 to 12, the feeding facility including an external reservoir spaced from the solid substrate (11).

15. The nebulizer according to any one of claims 1 to 14, further comprising: a housing, the housing defining a chamber and having an outlet to the chamber for the egress of atomised fluid particles from within the chamber, the solid substrate being within the chamber of the housing.

## Patentansprüche

1. Vernebler (10), umfassend:
- einem festen Substrat (11),
- einen linearen Kanal (12), der in dem festen Substrat (11) geformt ist und eine geschlossene Basis aufweist,
gegenüberliegende Seitenwände und eine Öffnung, die sich durch eine Oberseite (16) des festen Substrats erstreckt,
- einen Schwingungsgenerator (17), der an dem festen Substrat (11) an einer vom linearen Kanal (12) beabstandeten Position angebracht ist, um hochfrequente Schwingungen zu erzeugen, die durch das feste Substrat auf den linearen Kanal übertragen werden, um das Fluid innerhalb des linearen Kanals zu zerstäuben,
- eine Zuführvorrichtung zum Zuführen eines Fluids zu dem linearen Kanal (12),
**gekennzeichnet durch**
- die Oberfläche des linearen Kanals (12) so behandelt ist, dass sie hydrophil ist,
- den linearen Kanal (12) mit einem hohen Seitenverhältnis, wobei die Tiefe des linearen Kanals wesentlich größer ist als die Breite des linearen Kanals, wobei die Tiefe des linearen Kanals im Bereich von 10,5 Mikrometern für einen linearen Kanal (12) liegt, der ein Seitenverhältnis von 7:1 aufweist.
eine lineare Kanalöffnung von 1,5 Mikrometern und eine Tiefe von bis zu 300 Mikrometern für einen linearen Kanal (12), der ein Seitenverhältnis von 15:1 und eine lineare Kanalöffnung von 20 Mikrometern aufweist.

2. Vernebler nach Anspruch 1, wobei das Seitenverhältnis des linearen Kanals zwischen 7:1 und 15:1 liegt.

3. Vernebler nach Anspruch 1 oder 2, wobei die Öffnung des linearen Kanals (12) durch die obere Oberfläche (16) des festen Substrats eine Breite quer zur Länge aufweist, wobei die Breite 1,5, 3, 5, 10 oder 20 Mikrometer beträgt.

4. Vernebler nach einem der Ansprüche 1 bis 3, wobei das feste Substrat (11) ein starres Substrat ist, insbesondere aus Silizium geformt ist.

5. Vernebler nach einem der Ansprüche 1 bis 4, wobei die Oberfläche des linearen Kanals (12) behandelt wird, um sie durch Aufbringen von O2-Plasma auf die lineare Kanaloberfläche hydrophil zu machen.

6. Vernebler nach einem der Ansprüche 1 bis 5, einschließend mehrere lineare Kanäle (12), die in einer parallelen Anordnung oder senkrecht zueinander oder in einer T- oder L-Formation für zwei lineare Kanäle oder in einer C- oder quadratischen/rechteckigen Formation für drei oder vier lineare Kanäle geformt sind.

7. Vernebler nach einem der Ansprüche 1 bis 6, wobei der Schwingungsgenerator (17) ein piezoelektrischer Aktuator ist, der eine piezoelektrische Scheibe verwendet, die elektrisch betätigt oder angetrieben wird.

8. Vernebler nach Anspruch 7, wobei der piezoelektrische Aktuator Antriebsvibrationen in einem Frequenzbereich von 500 kHz bis 5 MHz oder von 5 MHz bis 20 MHz erzeugt.

9. Vernebler nach einem der Ansprüche 1 bis 8, wobei sich der lineare Kanal (12) vollständig über das feste Substrat (11) von einer Seite zur anderen erstreckt und durch die Seitenkanten des festen Substrats offen ist.

10. Vernebler nach einem der Ansprüche 1 bis 8, wobei sich ein Ende des linearen Kanals (12) durch eine Seitenkante des festen Substrats (11) öffnet und das andere, gegenüberliegende Ende innerhalb des festen Substrats endet.

11. Vernebler nach einem der Ansprüche 1 bis 8, wobei beide Enden des linearen Kanals (12) innerhalb des festen Substrats (11) enden.

12. Vernebler nach einem der Ansprüche 1 bis 11, wobei die Zuführeinrichtung einen Vorratsbehälter (15) aufweist, in den Flüssigkeit zugeführt wird, um anschließend in den linearen Kanal (12) zu gelangen.

13. Vernebler nach Anspruch 12, wobei der Vorratsbehälter (15) in dem festen Substrat (11) geformt ist.

14. Vernebler nach einem der Ansprüche 1 bis 12, wobei die Zuführvorrichtung einen externen Behälter einschließt, der vom festen Substrat (11) beabstandet ist.

15. Vernebler nach einem der Ansprüche 1 bis 14, ferner umfassend: ein Gehäuse, wobei das Gehäuse eine Kammer definiert und einen Auslass zur Kammer für den Austritt von zerstäubten Fluidpartikeln aus der Kammer aufweist, wobei sich das feste Substrat innerhalb der Kammer des Gehäuses befindet.

## Revendications

1. Nébuliseur (10) comprenant :
- un substrat solide (11),
- un canal linéaire (12) formé dans le substrat solide (11) qui possède une base fermée,
des parois latérales opposées et une ouverture qui traverse une surface supérieure (16) du substrat solide,
- un générateur de vibrations (17) fixé au substrat solide (11) à une position espacée du canal linéaire (12) pour générer des vibrations à haute fréquence qui se transmettent à travers le substrat solide jusqu'au canal linéaire afin d'atomiser le fluide à l'intérieur du canal linéaire,
- un dispositif d'alimentation pour alimenter le canal linéaire (12) en fluide,
**caractérisé par**
- la surface du canal linéaire (12) étant traitée pour la rendre hydrophile,
- le canal linéaire (12) ayant un rapport d'aspect élevé dans lequel la profondeur du canal linéaire est beaucoup plus grande que sa largeur, la profondeur du canal linéaire étant comprise dans une plage de 10,5 micromètres pour un canal linéaire (12) ayant un rapport d'aspect de 7:1
et une ouverture de canal linéaire de 1,5 micromètre et jusqu'à une profondeur de 300 micromètres pour un canal linéaire (12) qui a un rapport d'aspect de 15:1 et une ouverture de canal linéaire de 20 micromètres.

2. Nébuliseur selon la revendication 1, le rapport d'aspect du canal linéaire allant de 7:1 à 15:1.

3. Nébuliseur selon la revendication 1 ou 2, l'ouverture du canal linéaire (12) à travers la surface supérieure (16) du substrat solide ayant une largeur transversale à la longueur, la largeur étant de 1,5, 3, 5, 10 ou 20 micromètres.

4. Nébuliseur selon l'une quelconque des revendications 1 à 3, le substrat solide (11) étant un substrat rigide, en particulier formé de silicium.

5. Nébuliseur selon l'une quelconque des revendications 1 à 4, la surface du canal linéaire (12) étant traitée pour la rendre hydrophile par l'application de plasma 02 sur la surface du canal linéaire.

6. Nébuliseur selon l'une quelconque des revendications 1 à 5, comportant plusieurs canaux linéaires (12) formés dans un réseau parallèle ou perpendiculairement les uns aux autres, ou dans une formation en T ou en L pour deux canaux linéaires, ou dans une formation en C ou carrée/rectangulaire pour trois ou quatre canaux linéaires.

7. Nébuliseur selon l'une quelconque des revendications 1 à 6, le générateur de vibrations (17) étant un actionneur piézoélectrique qui utilise un disque piézoélectrique actionné ou excité électriquement.

8. Nébuliseur selon la revendication 7, l'actionneur piézoélectrique générant des vibrations d'actionnement dans une gamme de fréquences de 500 kHz à 5 MHz ou de 5 MHz à 20 MHz.

9. Nébuliseur selon l'une quelconque des revendications 1 à 8, le canal linéaire (12) s'étendant entièrement à travers le substrat solide (11) d'un côté à l'autre et ouvert à travers les bords latéraux du substrat solide.

10. Nébuliseur selon l'une quelconque des revendications 1 à 8, une extrémité du canal linéaire (12) s'ouvrant à travers un bord latéral du substrat solide (11) et l'autre extrémité opposée se terminant à l'intérieur du substrat solide.

11. Nébuliseur selon l'une quelconque des revendications 1 à 8, les deux extrémités du canal linéaire (12) se terminant à l'intérieur du substrat solide (11).

12. Nébuliseur selon l'une quelconque des revendications 1 à 11, le dispositif d'alimentation ayant un réservoir (15) dans lequel le fluide est introduit pour un passage ou un acheminement ultérieur dans le canal linéaire (12).

13. Nébuliseur selon la revendication 12, le réservoir (15) étant formé dans le substrat solide (11).

14. Nébuliseur selon l'une quelconque des revendications 1 à 12, le dispositif d'alimentation comportant un réservoir externe espacé du substrat solide (11).

15. Nébuliseur selon l'une quelconque des revendications 1 à 14, comprenant en outre : un boîtier, le boîtier définissant une chambre et ayant une sortie vers la chambre pour la sortie des particules de fluide atomisées de l'intérieur de la chambre, le substrat solide étant à l'intérieur de la chambre du boîtier.
